# NEW EUROPEAN PATENT SPECIFICATION

(11) **EP 1 369 083 B2**
(45) Date of publication and mention of the opposition decision: **30.05.2012**
(45) Mention of the grant of the patent: 14.12.2005
(21) Application number: 03253439.8
(22) Date of filing: 02.06.2003
(51) Int. Cl.: A61B 5/15

(54) **Test strip container system**
Vorratsbehältnis für Teststreifen
Recipient de stockage pour bandes de test

(30) Priority: 03.06.2002 US 162245
(43) Date of publication of application: 10.12.2003
(73) Proprietor: LifeScan, Inc., Milpitas, CA 95035-6312 (US)
(72) Inventor: McAllister, Devin, Shrewsbury, Massachusetts 01545 (US); Leong, Koon-wah, Sunnyvale, California (US)
(74) Representative: Mercer, Christopher Paul

(56) References cited:
- EP-A- 0 985 376
- EP-A1- 1 285 629
- EP-A2- 0 951 939
- EP-A2- 1 174 083
- EP-A2- 1 203 563
- EP-B1- 0 901 634
- WO-A-01/00090
- WO-A-2002/036010
- DE-B- 2 803 345
- US- - 5 510 226
- US-A- 5 035 704
- US-A- 5 231 993
- US-A- 5 591 139
- US-A- 6 036 924

## Description

### FIELD OF THE INVENTION

The invention relates to the manner of storing, dispensing and disposing of analyte test strips.

### BACKGROUND OF THE INVENTION

Analyte concentration determination in physiological samples is of ever increasing importance to today's society. Such assays find use in a variety of application settings, including clinical laboratory testing, home testing, *etc.,* where the results of such testing play a prominent role in the diagnosis and management of a variety of disease conditions. Analytes of interest include glucose for diabetes management, cholesterol for monitoring cardiovascular conditions, and the like. In response to this growing importance of analyte concentration determination, a variety of analyte concentration determination protocols and devices for both clinical and home testing have been developed.

In determining the concentration of an analyte in a physiological sample, a physiological sample must first be obtained. Obtaining the sample often involves cumbersome and complicated devices which may not be easy to use or may be costly to manufacture. The analyte concentration determination process may also involve a multitude of steps. First, a sample is accessed by use of a skin-piercing mechanism, *e.g.*, a needle or lancet, which accessing may also involve the use of a sample collection mechanism, *e.g.*, a capillary tube. Next, the sample must then be transferred to a testing device, *e.g.*, a test strip or the like, and then oftentimes the test strip is then transferred to a measuring device such as a meter. Thus, the steps of accessing the sample, collecting the sample, transferring the sample to a biosensor, and measuring the analyte concentration in the sample are often performed as separate, consecutive steps with various device and instrumentation.

Because of these disadvantages, it is not uncommon for patients who require frequent monitoring of an analyte to simply become non-compliant in monitoring themselves. With diabetics, for example, the failure to measure their glucose level on a prescribed basis results in a lack of information necessary to properly control the level of glucose. Uncontrolled glucose levels can be very dangerous and even life threatening.

Numerous approaches have been developed to facilitate test regiment compliance or simplify testing for users. Certain devices combine a lancing-type device with various other components involved in the analyte concentration determination procedure in order to simplify the assay process. For example, U.S. Patent No. 6,099,484 discloses a sampling device which includes a needle associated with a spring mechanism, a capillary tube associated with a pusher, and a test strip. U.S. Patent No. 5,820,570 discloses an apparatus which includes a base having a hollow needle and a cover having a membrane, whereby the base and cover are connected together at a hinge point. Still further, U.S. Patent Application Atty. Docket No. 054, entitled "Minimal Procedure Analyte Test System," teaches a system and discusses other systems combining lancing device, meter and test strip handling functionality.

Other systems, such as described in U.S. Patent Nos. 5,510,266; 5,575,403 and 5, 863,800 offer test strip storage and dispensing system, but no integrally lancing features. The devices described in the '403 patent does, however, include meter functionality.

With respect to each of these systems, provision is made for storing test strips in an isolated manner and then conveniently dispensing them from their individual packaging. Other systems in which test strips are individually sealed in an aluminum laminate foil are known as well. To remove the test elements, the user tears open the foil and takes out the test element.

Simply sealing test strip elements in foil has several drawbacks. Sealing test elements in aluminum laminates produces a lot of waste material and requires a user to tear or puncture the aluminum laminate, often causing problems in view of the dexterity required, which ill people often face. In the '266, '403 and '800 patents, since test elements are provided in a single container with multiple compartments, signification waste packaging issues are avoided. Dexterity challenges are also addressed by providing collateral hardware for extracting the test strips from their packaging. In each of the references, the test strips provided are pushed out of the packages sensor-side first In the '266 and '403 patents the face or front of the test strip itself punches through a foil covering.

Similar containers comprising lancets and test ships are disclosed in document US 6036924, which discloses a container for storing test ships including a lancet and comprising a receptacle with an access aperture and a sheath portion.

Nether of these systems is amenable to use with test strips including integral (forward-facing) lance features as are the storage containers of the present invention. The mode of operation of the systems described in the '266 and '800 patents would destroy delicate microneedle features.

Further, while the approach in the '403 patent could possibly be adapted for use with a test element that integrally includes lance features, the planar storage orientation of test strips contemplated therein is not space efficient and requires complex movement of members (including the test strips held in covered wells) in use. Aspects of the present invention offer a more elegant approach with a concomitant reduction in cost of the storage devices or meter/lancing devices that may be used with the same.

Another aspect of the present invention is geared toward yet another consideration in handling test strips, *i*.*e*., test strip disposal. PCT publication WO 01/23885 provides for a test strip dispensing system with an integral waste disposal section. However, no apparent provision is made for isolating used tester portions, contaminated with biological fluid from unused members. Furthermore, individual test strip portions are not isolated. Consequently, exposure of one portion results in exposure of others, thereby introducing moisture or contaminates which can have a deleterious effect on test strip reagent compounds and needle sterility, respectively.

EP 1 285 629 A1 which comprises prior art under Art.54(3) EPC for DE, FR, GB and IT only, discloses a test strip container system as recited in claim 1 for all designated contracting states except DE, FR, GB and IT.

EP 0 951 939 A2 discloses a test strip container system comprising: a plurality of test strips, each including face or shoulder portions; a container body defining a plurality of test strip receptacles, each receptacle having an access aperture at one end and at least one ledge for supporting a test strip at its face or shoulder portions; a barrier portion for closing off at least some of said access apertures at said one end such that said test strips can be exposed for use one at a time; each receptacle having: an inset portion extending from said at least one ledge to form a sheath portion at another end, and an access path to the test strip from its rear via said access aperture.

EP 0 901 634 B1 discloses a test strip container system comprising: a container body, said container body defining a plurality of test strip receptacles at one end, and a barrier portion for closing off at least some of said test strip receptacles.

The present invention offers further improvement in test strip handling and use. Each aspect of the invention addresses certain concerns, thus providing a system better able to meet the public's needs.

### SUMMARY OF THE INVENTION

The present invention provides test strip container systems as recited in the claims.

Each variation of the invention includes a test strip container adapted to individually receive a plurality of test strips in a sealed fashion. The type of seal used affords the ability to expose test strips for use one at a time. Preferred seals for such purpose include foil (such as an aluminum foil laminate) and a rotatable lid or cap including a single port to be moved from one test strip receptacle portion to the next.

The test strip receptacle is configured to receive and protect a lance member including at least one forward-facing microneedle. This adaptation or configuration includes an inset portion to house the needle and an access path to the test strip from its rear.

The inventive container may also include a waste receptacle that can be closed-off for safe storage of spent test strips apart from unused members. Since protecting used text strips held in the waste receptacle from exposure is not critical, any sort of cap or closure mechanism may be employed in this regard. In preferred variations of the invention, however, where a cylindrical container body is employed the waste receptacle is situated across from or on the opposite side from where unused test strips are accessed. Various optional safety features may be provided in connection with the waste receptacle.

In use, the container preferably interfaces with a meter/lancing device to select and/or retrieve a test strip. A user may intermittently position the container to facilitate such action or load the container into the meter/lancing device.

### BRIEF DESCRIPTION OF THE DRAWINGS

Each of the following figures diagrammatically illustrates aspects of the present invention. Variation of the invention from that shown in the figures is contemplated.

Figure 1 is a perspective view of a representative meter and test strip as may be used in connection with variations of the present invention.

Figures 2A and 2B are perspective views of variations of the inventive container.

Figure 3 is a perspective view of a section of a receptacle portion of the container, located as depicted in FIG 2B, together with a test strip indicating how it is placed in the same.

Figures 4A-4C are perspective views of a variation of the inventive container incorporating a waste receptacle.

### DETAILED DESCRIPTiON OF THE INVENTION

In describing the invention in greater detail than provided in the Summary above, colorimetric and electrochemical test strips sensors are first described, followed by discussion of features and the use of exemplary combination test strip meter and lancing device of the present invention. After this background discussion, features of the invention containers are described in detail. Finally, kits advantageously incorporating components of the present invention are described.

Before the present invention is described in such detail, however, it is to be understood that this invention is not limited to particular variations set forth and may, of course, vary. Various changes may be made to the invention described and equivalents may be substituted without departing from the true spirit and scope of the invention. In addition, many modifications may be made to adapt a particular situation, material, composition of matter, process, process act(s) or step(s), to the objective(s), spirit or scope of the present invention. All such modifications are intended to be within the scope of the claims made herein. For example, description of the use of electrochemical and photometric sensor type test strips is not intended to be limiting; those skilled in the art will appreciate that the subject devices, systems and methods are useful in the measurement of other physical and chemical characteristics of biological substances, *e.g.*, blood coagulation time, blood cholesterol level, *etc*.

Methods recited herein may be carried out in any order of the recited events which is logically possible, as well as the recited order of events. Furthermore, where a range of values is provided, it is understood that every intervening value, between the upper and lower limit of that range and any other stated or intervening value in that stated range is encompassed within the invention. Also, it is contemplated that any optional feature of the inventive variations described may be set forth and claimed independently, or in combination with any one or more of the features described herein.

The referenced items are provided solely for their disclosure prior to the filing date of the present application. Nothing herein is to be construed as an admission that the present invention is not entitled to antedate such material by virtue of prior invention.

Reference to a singular item, includes the possibility that there are plural of the same items present. More specifically, as used herein and in the appended claims, the singular forms "a," "and," "said" and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Finally, it is noted that unless defined otherwise herein, all technical and scientific terms used herein have the same meaning as commonly understood by one of ordinary skill in the art to which this invention belongs.

### Colorimetric/Photometric Sensor Variations

In testers including colorimetric or photometric (herein used interchangeably) biosensor, the same is provided by at least a matrix and/or a membrane for receiving a sample and a reagent composition (set within the matrix or membrane) set upon a support structure. Where a membrane as well as a matrix is provided, the membrane will generally be placed opposite of the support structure upon the matrix. A membrane advantageously includes apertures or pores for sample access.

In some embodiments, the sensor comprises a membrane containing a reagent composition impregnated therein while a matrix may or may not contain reagent composition. Often the matrix preferably provides a deposition area for the various members of the signal producing system, described *infra*, as well as for the light absorbing or chromogenic product produced by the signal producing system, *i.e.*, the indicator, as well as provides a location for the detection of the light-absorbing product produced by the indicator of the signal producing system .

A membrane provided may comprise a membrane that exhibits aqueous fluid flow properties and is sufficiently porous (*i.e.,* provides sufficient void space) for chemical reactions of a signal producing system to take place. Ideally, the membrane pore structure would not support red blood cell flow to the surface of the membrane being interrogated (*i.e.,* the color intensity of which is a subject of the measurement correlated to analyte concentration). Any matrix provided may or may not have pores and/or a porosity gradient, *e.g.* with larger pores near or at the sample application region and smaller pores at the detection region.

Materials from which a membrane may be fabricated vary, include polymers, *e.g.* polysulfone, polyamides, cellulose or absorbent paper, and the like, where the material may or may not be functionalized to provide for covalent or non-covalent attachment of the various members of the signal producing system. In a tester made a thin membrane material, the tester may require less than 1/2 µl of sample to wet a sufficiently large area of the membrane to obtain a good optical measurement.

Regarding suitable matrices, a number of different types have been developed for use in various analyte detection assays, which matrices may differ in terms of materials, dimensions and the like, where representative matrices include, but are not limited to, those described in U.S. Patent Nos.: 4,734,360; 4,900,666; 4,935,346; 5,059,394; 5,304,468; 5,306,623; 5,418,142; 5,426,032; 5,515,170; 5,526,120; 5,563,042; 5,620,863; 5,753,429; 5,573,452; 5,780,304; 5,789,255; 5,843,691; 5,846,486; 5,968,836 and 5,972,294.

However configured, one or more members of a signal producing system of the biosensor produce a detectable product in response to the presence of analyte, which detectable product can be used to derive the amount of analyte present in the assayed sample. In the subject test strips, the one or more members of the signal producing system are preferably associated with (*e.g.*, covalently or non-covalently attached to) at least a portion of (*i.e.,* the detection region) the matrix or membrane, and in many embodiments to substantially all of the same.

The signal producing system may comprise an analyte oxidation signal producing system. By analyte oxidation signal producing system, it is meant that in generating the detectable signal from which the analyte concentration in the sample is derived, the analyte is oxidized by a suitable enzyme to produce an oxidized form of the analyte and a corresponding or proportional amount of hydrogen peroxide. The hydrogen peroxide is then employed, in turn, to generate the detectable product from one or more indicator compounds, where the amount of detectable product generated by the signal measuring system, *i.e.* the signal, is then related to the amount of analyte in the initial sample. As such, the analyte oxidation signal producing systems present in the subject test strips are also correctly characterized as hydrogen peroxide based signal producing systems.

Hydrogen peroxide based signal producing systems include an enzyme that oxidizes the analyte and produces a corresponding amount of hydrogen peroxide, where by corresponding amount is meant that the amount of hydrogen peroxide that is produced is proportional to the amount of analyte present in the sample. The specific nature of this first enzyme necessarily depends on the nature of the analyte being assayed but is generally an oxidase or dehydrogenase. As such, the first enzyme may be: glucose oxidase (where the analyte is glucose), or glucose dehydrogenase either using NAD or PQQ as cofactor; cholesterol oxidase (where the analyte is cholesterol); alcohol oxidase (where the analyte is alcohol); lactate oxidase (where the analyte is lactate) and the like. Other oxidizing enzymes for use with these and other analytes of interest are known to those skilled in the art and may also be employed. In those preferred embodiments where the reagent test strip is designed for the detection of glucose concentration, the first enzyme is glucose oxidase. The glucose oxidase may be obtained from any convenient source (*e.g.* a naturally occurring source such as Aspergillus niger or Penicillum, or recombinantly produced).

The second enzyme of such a signal producing system is an enzyme that catalyzes the conversion of one or more indicator compounds into a detectable product in the presence of hydrogen peroxide, where the amount of detectable product that is produced by this reaction is proportional to the amount of hydrogen peroxide that is present. This second enzyme is generally a peroxidase, where suitable peroxidases include: horseradish peroxidase (HRP), soy peroxidase, recombinantly produced peroxidase and synthetic analogs having peroxidative activity and the like. See, *e.g.*, Y. Ci, F. Wang; Analytica Chimica Acta, 233 (1990), 299-302.

Indicator compound or compounds provided are preferably ones that are either formed or decomposed by the hydrogen peroxide in the presence of the peroxidase to produce an indicator dye that absorbs light in a predetermined wavelength range. Preferably the indicator dye absorbs strongly at a wavelength different from that at which the sample or the testing reagent absorbs strongly. The oxidized form of the indicator may be a colored, faintly-colored, or colorless final product that evidences a change in color of the testing side of the membrane. That is to say, the testing reagent can indicate the presence of glucose in a sample by a colored area being bleached or, alternatively, by a colorless area developing color.

Indicator compounds that are useful in the present invention include both one-and two-component chromogenic substrates. One-component systems include aromatic amines, aromatic alcohols, azines, and benzidines, such as tetramethyl benzidine-HCl. Suitable two-component systems include those in which one component is MBTH, an MBTH derivative (see *e.g.*, those disclosed in EP-A-0 781 350), or 4-aminoantipyrine and the other component is an aromatic amine, aromatic alcohol, conjugated amine, conjugated alcohol or aromatic or aliphatic aldehyde. Exemplary two-component systems are 3-methyl-2-benzothiazolinone hydrazone hydrochloride (MBTH) combined with 3-dimethylaminobenzoic acid (DMAB); MBTH combined with 3,5-dichloro-2-hydroxybenzene-sulfonic acid (DCHBS); and 3-methyl-2-benzothiazolinone hydrazone N-sulfonyl benzenesulfonate monosodium (MBTHSB) combined with 8-anilino-1 naphthalene sulfonic acid ammonium (ANS). In certain embodiments, the dye couple MBTHSB-ANS is preferred.

In yet other embodiments of colorimetric sensors that may be used in the present invention, signal producing systems that form a fluorescent detectable product (or detectable non- fluorescent substance, *e.g.* in a fluorescent background) may be employed, such as those described in Kiyoshi Zaitsu, Yosuke Ohkura, "New fluorogenic substrates for Horseradish Peroxidase: rapid and sensitive assay for hydrogen peroxide and the Peroxidase", Analytical Biochemistry (1980) 109, 109-113. Examples of such colorimetric reagent test strips suitable for use with the subject invention include those described in U.S. Patent Nos. 5,563,042; 5,753,452; 5,789,255.

### Electrochemical Sensor Variations

Instead of using a colorimetric sensor as described above, test strips used in connection with the present invention may employ an electrochemical sensor. . Typically, an electrochemical sensor comprises at least a pair of opposing electrodes, although electrochemical test strips with planar electrodes may be used in the prevent invention.

Where opposing-electrode type strips are employed, at least the surfaces of electrodes facing each other are comprised of a conductive layer such as a metal, where metals of interest include palladium, gold, platinum, silver, iridium, stainless steel and the like as well as carbon (conductive carbon ink) and indium doped tin oxide.

One conductive layer is preferably formed by sputtering a thin layer of gold (Au), the other by sputtering a thin layer of palladium (Pd). Alternately, the electrodes may be formed by screen printing a selected conductive pattern, including conductive leads, with a carbon or metal ink on the backing surfaces. An additional insulating layer may be printed on top of this conductive layer which exposes a precisely defined pattern of electrodes. However formed, after deposition of conductive layers, the surface may be subsequently treated with a hydrophilic agent to facilitate transport of a fluid sample into the reaction zone there between. Depending on the voltage sequence applied to the cell, one electrode may serve as a counter/reference electrode and the other as the working electrode of the electrochemical cell. However, where a double pulse voltage waveform is employed, each electrode acts as a counter/reference and working electrode once during analyte concentration measurement.

Regardless of reaction zone or electrode configuration, a reagent coating is typically provided therein. Reagent systems of interest typically include an enzyme and a redox active component (mediator). The redox component of the reagent composition, when present, is made up of one or more redox agents. A variety of different redox agents (*i.e.*, mediators) are known in the art and include: ferricyanide, phenazine ethosulphate, phenazine methosulfate, pheylenediamine, 1-methoxyphenazine methosulfate, 2,6-dimethyl-1,4-benzoquinone, 2,5-dichloro-1,4-benzoquinone, ferrocene derivatives, osmium bipyridyl complexes, ruthenium complexes, and the like. In many embodiments, the redox active component of particular interest is ferricyanide, and the like. The enzyme of choice may vary depending on the analyte concentration which is to be measured. For example, suitable enzymes for the assay of glucose in whole blood include glucose oxidase or dehydrogenase (NAD or PQQ based). Suitable enzymes for the assay of cholesterol in whole blood include cholesterol oxidase and esterase.

Other reagents that may be present in the reaction area include buffering agents (*e.g.*, citraconate, citrate, malic, maleic, phosphate, "Good" buffers and the like); divalent cations (*e.g.*, calcium chloride, and magnesium chloride); surfactants (*e.g.*, Triton, Macol, Tetronic, Silwet, Zonyl, Aerosol, Geropon, Chaps, and Pluronic); and stabilizing agents (*e.g.,* albumin, sucrose, trehalose, mannitol and lactose).

Examples of electrochemical biosensors suitable for use with the subject invention include those described in EP-A-1 067 384, EP-A-1 252 514, EP-A-1 254 365, WO 02/48707 and WO 02/50609.

### Test Strip Systems and Use

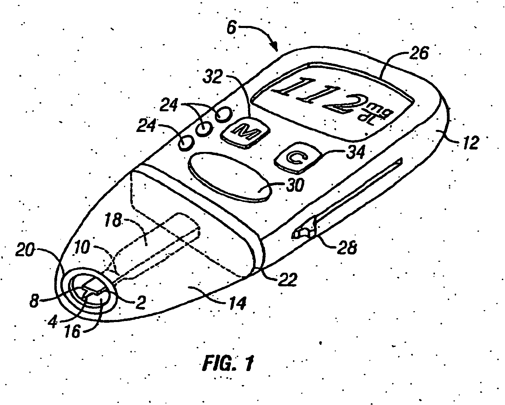

As mentioned above, the test strips housed in the container as described further below is preferably used an automated lancing and meter device. FIG 1 shows one such device.

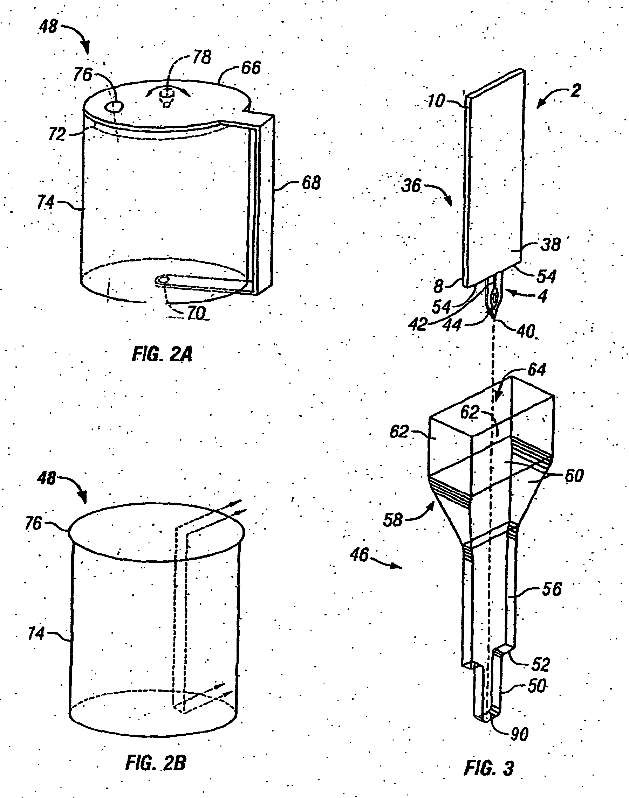

A test strip **2**, including a skin-piercing element **4** is set within a meter **6**. The test strip includes a biosensor (hidden from view) adjacent to a flow path of the needle **4**. Preferably, the sensor is of the sort described above. Referring to FIG 3, test strip device **2** has a first end **8** and a second end **10**, wherein the skin-piercing or lancing blade or needle **4** is associated with first end **8** and at least the second end **10** is configured for insertion into meter **6**. Further constructional details or options for test strip 2 may be as described in EP-A-1 281 352, EP 1360931 and EP 1360933.

Regarding meter **6,** it preferably has an ergonomically-designed housing **12** having dimensions which allow it to be comfortably held and manipulated with one hand. Housing **12** may be made of a metal, plastic or other suitable material, preferably one that is light weight but sufficiently durable. The distal portion **14** of the housing provides an aperture **16** through which test strip device **2** is advanced from a retracted position within meter **6** to an extended position wherein at least a portion of the test strip microneedle/lancet **4** extends a distance outside aperture **16.**

Distal portion **14** further defines a chamber in which test strip device **2** is received within a test strip receiving mechanism **18.** Test strip device **2** may be inserted into meter **6** by removing distal housing portion **14** from housing **12** and inserting test strip device **2** into test strip receiving mechanism **18.** Alternatively, test strip device **2** may be inserted into meter **6** and received into mechanism **18** via aperture **14.**

Preferably, distal housing portion **14** is transparent or semi-transparent to allow the user to visually confirm proper engagement between test strip device **2** and receiving area **18** prior to conducting the analyte concentration assay, as well as to visualize the test site and to visually confirm the filling of strip **2** with body fluid during the assay (especially if electronic sensing is not provided to discern the same) . When test strip device **2** is properly seated within receiving mechanism **18,** the biosensor with test strip device **2** operatively engages with the meter's testing components. In the case of electrochemical test strip embodiments, the electrodes of the biosensor operatively engage with the meter's electronics; with colorimetric test strip embodiments, the matrix or membrane area having a signal producing system is operatively aligned with the meter's optical components. The meter's electronics or optical componentry, upon sensing when the reaction zone or matrix area, respectively, within test strip device **2** is filled with the sampled fluid, supplies an input signal to the test strip biosensor and receives an output signal therefrom which is representative of the sample fluid characteristic being measured.

Circumferentially positioned about aperture **16** is a pressure ring **20,** the distal surface of which is applied to the skin and encircles the piercing site within the skin during a testing procedure. The compressive pressure exerted on the skin by pressure ring **20** facilitates the extraction of body fluids from the surrounding tissue and the transfer of such fluid into test strip device **2.**

Distal housing portion **14** is preferably itself in movable engagement with meter **6** wherein distal housing portion **14** is slightly translatable or depressible along a longitudinal axis of the meter. Between distal housing portion **14** and the a proximal portion of housing **12**, is a pressure sensor **22** which senses and gauges the amount of pressure exerted on distal housing portion **14** when compressing pressure ring **20** against the skin. Pressure sensor **22** is preferably an electrical type sensor which may be of the kind commonly known in the field of electronics. Pressure sensor indicators **24,** in electrical communication with pressure sensor **22,** are Provided to indicate the level of pressure being applied to distal housing portion **14** so that the user may adjust the amount of pressure being applied, if necessary, in order to apply an optimal pressure.

In many embodiments, meter **6** has a display **26,** such as an LCD display, for displaying data, such as input parameters and test results. Additionally, meter **6** has various controls and buttons for inputting data to the meter's processing components and for controlling the piercing action of test strip device **2.** For example, lever **28** is used to retract test strip device **2** to a loaded position within meter **6** and thereby preload a spring mechanism (not shown) for later, on-demand extension or ejection of test strip device **2** from aperture **16** by depressing button **30**. When distal housing portion **14** is properly positioned on the skin, such ejection of test strip device **2** causes microneedle **4** to instantaneously pierce the skin for accessing the body fluid therein. Buttons **32** and **34,** when depressed, input signals to the meter's processing components indicating whether the measurement to be made is for testing/information purposes (and for recovering the test results from a memory means within the meter's electronics) or for calibration purposes, respectively.

Meter **6** may further be configured to receive and retain a replaceable cartridge containing a plurality of the subject test strip devices. After using a test strip device, the meter may either eject the used test strip from the meter or store them for disposal at a later time. Such a configuration eliminates the necessary handling of test strips, thereby minimizing the likelihood of damage to the strip and inadvertent injury to the patient. Furthermore, because manual handling of the test strips is eliminated, the test strips may be made much smaller thereby reducing the amount of materials required, providing a cost savings. The meter disclosed in EP 1 362 551 is of particular relevance in regard to these considerations.

Additionally, certain aspects of the functionality of meters suitable for use with the subject systems are disclosed in U.S. Patent No. 6,193,873, as well as in EP-A-1 252 514, EP-A-1 252 365, WO 02/48707, WO02/50609 and EP-A-1 284 121. Of course, in those embodiments using a colorimetric assay system, a spectrophotometer or optical meter will be employed, where certain aspects of the functionality of such meters suitable for use are described in, for example, U.S. Patent Nos. 4,734,360, 4;900,666, 4,935,346; 5,059,394, 5,304,468, 5,306,623, 5,418,142, 5,426,032, 5,515,170, 5,526,120, 5,563,042, 5,620,863, 5,753,429, 5,773,452, 5,780,304, 5,789,255, 5,843,691, 5,846,486, 5,968,836 and 5,972,294.

In use, the subject invention provides methods for determining a characteristic of the sample, *e.g.*, the concentration of an analyte in a sample. The subject methods find use in the determination of a variety of different analyte concentrations, where representative analytes include glucose, cholesterol, lactate, alcohol, and the like. In many embodiments, the subject methods are employed to determine the glucose concentration in a physiological sample. Test strip devices **2** used in connection with the present invention are particularly suited for use in determining the concentration of an analyte in blood or blood fractions, and more particularly in whole blood or interstitial fluid.

In using test strip **2,** meter **6** is actuated so microneedle **4** is inserted into a target area of skin. Typically, the skin-piercing element is inserted into the skin of a finger or forearm for about 1 to 60 seconds, usually for about 1 to 15 seconds and more usually for about 1 to 5 seconds. Depending on the type of physiological sample to be obtained, the subject skin-piercing element **4** may be penetrated to various skin layers, including the dermis, epidermis and the stratum corneum, but in many embodiments will penetrate no farther than the subcutaneous layer of the skin.

The test strips is preferably loaded into the meter automatically by way of the meter interfacing with a cartridge or container as described further below. Interface member **18** may simply be a device that captures and holds test strip or it may include electrode elements (particularly for use in interfacing with electrochemical test strips).

Once test strip device **2** is properly received within mechanism **18,** it may then be spring loaded or cocked by means of lever **28,** thereby retracting the test strip device **2** and preparing it for firing Meter **6** is then positioned substantially perpendicular to the targeted skin surface wherein distal housing portion **14,** and more specifically pressure ring **20,** is caused to contact the target skin area Some compressive pressure may be manually applied to the target skin area, *i.e.,* by pressing the distal end of meter **14** against the target skin area, to ensure that skin-piercing element **4** is properly inserted into the skin. By applying such pressure, a counter force causes distal housing portion **14** to press back upon pressure sensor **22.**

The relative amount (*i.e.,* high, normal and low) of counter pressure is then measured and displayed by optional pressure sensor indicators **24.** Preferably, the amount of pressure applied should generally be in the "normal" range. Indicators **24** inform the user as to when too much or too little pressure is being applied. When the indicators show that the applied pressure is "normal", the user may then depress the spring-release button **30**. Due to the spring force released, receiving/carrying mechanism **18** and test strip device **2** are caused to thrust forward thereby causing skin-piercing element **4** to extend from aperture **16** and puncture the targeted skin area.

The penetration of skin-piercing element **4** into the skin preferably create a fluid sample pooling area (defined by the recess or opening within skin-piercing element shown in FIG 3). In which case, sample fluid enters the pooling area by the open-space configuration (*e.g.*, recess or opening, within skin piercing element **4**), and possibly also from the opposite side of the skin-piercing element. The pooled sample fluid is then transferred directly to the reaction zone of a test strip or thereto by a fluid pathway by at least a capillary force exerted on the pooled fluid. Where no enlarged pooling area is provided, a simple capillary channel may prove effective in certain situations as well, though such a set-up may not be most preferred.

In any case, the transfer of fluid from the wound site to the biosensor may be further facilitated by exerting physical positive pressure circumferentially around the penetration site by means of a pressure ring **20** or by applying a source of negative pressure through the fluid channel thereby vacuuming the body fluid exposed to the distal end of the channel. Fluid passing into the biosensor reaction zone may simply fill the area or alternately be distributed by subchannels or another similar distribution feature.

Once meter **6** senses that the reaction zone or matrix area is completely filled with the sample of body fluid, the meter electronics or optics are activated to perform analysis of the extracted sample. At this point, the meter may be removed by the patient from the penetration site or kept on the skin surface until the test results are shown on the display. Meter **6** may alternatively or additionally include means for automatically retracting the microneedle strip from the skin once the reaction cell is filled with the body fluid sample.

With an electrochemical-based analyte concentration determination assay, an electrochemical measurement is made using the counter/reference and working electrodes. The electrochemical measurement that is made may vary depending on the particular nature of the assay and the meter with which the electrochemical test strip is employed, (*e.g.*, depending on whether the assay is coulometric, amperometric or potentiometric). Generally, the electrochemical measurement will measure charge (coulometric), current (amperometric) or potential (potentiometric), usually over a given period of time following sample introduction into the reaction area. Methods for making the above described electrochemical measurement are further described in U.S. Patent Nos: 4,224,125; 4,545,382; and 5,266,179; as well as in International Patent Publications WO 97/18465 and WO 99/49307.

Following detection of the electrochemical signal generated in the reaction zone, the amount of the analyte present in the sample is typically determined by relating the electrochemical signal generated from a series of previously obtained control or standard values. In many embodiments, the electrochemical signal measurement steps and analyte concentration derivation steps, are performed automatically by a device designed to work with the test strip to produce a value of analyte concentration in a sample applied to the test strip. A representative reading device for automatically practicing these steps, such that user need only apply sample to the reaction zone and then read the final analyte concentration result from the device, is further described in EP-A-1 067 384.

For a colorimetric or photometric analyte concentration determination assay, sample applied to a subject test strip, more specifically to a reaction area of a test strip, is allowed to react with members of a signal producing system present in the reaction zone to produce a detectable product that is representative of the analyte of interest in an amount proportional to the initial amount of analyte present in the sample. The amount of detectable product (*i.e.,* signal produced by the signal producing system) is then determined and related to the amount of analyte in the initial sample. With such colorimetric assays, optical-type meters are used to perform the above mentioned detection and relation steps. The above described reaction, detection and relating steps, as well as instruments for performing the same, are further described in U.S. PatentNos. 4,734,360; 4,900,666; 4,935,346; 5,059,394; 5,304,468; 5,306,623; 5,418,142; 5,426,032; 5,515,170; 5,526,120; 5,563,042; 5,620,863; 5,753,429; 5,773,452; 5,780,304; 5,789,255; 5,843,691; 5,846,486; 5,968,836 and 5,972,294. Examples of such colorimetric or photometric reagent test strips suitable for use with the subject invention include those described in U.S. Patent Nos.: 5,563,042; 5,753,452; 5,789,255.

### Container and Test Strip Devices/Combinations

FIG. 3 shows a test strip as introduced above in. A strip or substrate portion **36** carries the biosensor **38,** usually adjacent needle **4.** Each test strip preferably includes at least one lancet/needle or skin piercing element **4,** typically having a pointed tip **40.** In addition the body of lance **4** may incorporate various features to collect and/or convey a biological sample to a given test strip sensor.

Actually, any suitable shape of skin-piercing element **4** may be employed with the subject test strip devices, as long as the shape enables the skin to be pierced with minimal pain to the patient. For example, the skin-piercing element may have a substantially flat or planar configuration, or may be substantially cylindrical-like, wedge-like or triangular in shape such as a substantially flattened triangle-like configuration, blade-shaped, or have any other suitable shape. The cross-sectional shape of the skin-piercing element, or at least the portion of skin-piercing element that is penetrable into the skin, may be any suitable shape, including, but not limited to, substantially rectangular, oblong, square, oval, circular, diamond, triangular, star, *etc.* Additionally, the skin-piercing element may be tapered or may otherwise define a point or apex at its distal end. Such a configuration may take the form of an oblique angle at the tip or a pyramid or triangular shape or the like.

The dimensions of the skin-piercing element may vary depending on a variety of factors such as the type of physiological sample to be obtained, the desired penetration depth and the thickness of the skin layers of the particular patient being tested. Generally, the skin-piercing element is constructed to provide skin-piercing and fluid extraction functions and, thus, is designed to be sufficiently robust to withstand insertion into and withdrawal from the skin. Typically, to accomplish these goals, the ratio of the penetration length (defined by the distance between the base of the skin-piercing element and its distal tip) to diameter (where such diameter is measured at the base of the skin-piercing element) is from about 1 to 1, usually about 2 to 1, more usually about 5 to 1 or 10 to 1 and oftentimes 50 to 1.

The total length of the skin-piercing elements generally ranges from about 1 to 30,000 microns, usually from about 100 to 10,000 microns and more usually from about 1,000 to 3,000 microns. The penetration length of the skin-piercing elements generally ranges from about 1 to 5000 microns, usually about 100 to 3000 microns and more usually about 1000 to 2000 microns. The height or thickness of skin-piercing elements, at least the thickness of the distal portion 4, typically ranges from about 1 to 1000 microns, usually from about 10 to 500 microns and more usually from about 50 to 250 microns. The outer diameter at the base generally ranges from about 1 to 2000 microns, usually about 300 to 1000 microns and more usually from about 500 to 1000 microns. In many embodiments, the outer diameter of the distal tip generally does not exceed about 100 microns and is generally less than about 20 microns and more typically less than about 1 micron. However, it will be appreciated by one of skill in the art that the outer diameter of the skin-piercing element may vary along its length or may be substantially constant.

Regarding the fluid-conveying features noted that be incorporated in lance element **4,** a channel **42,** preferably of capillary dimensions may be provided. In addition (or alternately), a recessed pooling area or section **44** may be provided. Such a recessed or space-defining area is used to create a space or volume within the pierced tissue. This space serves as a reservoir within which bodily fluid is caused to pool *in situ* prior to being transferred to the biosensor portion of the subject test strip devices. As such, the availability of a greater volume of body fluid can be provided with a tip that is smaller and/or sharper than conventional microneedles, thereby reducing pain. The greater availability of body fluid also results in a faster collection rate of sampling.

Generally, space-defining lancet configurations in the present invention create or define a space within the pierced tissue having a volume at least as great as the available fluid volume in the reaction zone of the biosensor. Such space or volume ranges from about 10 to 1,000 nL, and more usually from about 50 to 250 nL. Such volume occupies a substantial portion of the entire volume occupied by the structure of the skin-piercing element, and ranges from about 50% to 99% and more usually from about 50% to 75% of the entire volume occupied by the skin piercing element.

While not shown, the test strip may include secondary fluid transfer pathways set in fluid communication with channel **42** to convey sample outwardly, dispersing the same across the sensor employed in an opposing, attached test strip. Like channel **42**, secondary pathways or channels are preferably dimensioned so as to exert a capillary force on fluid within the pooling area defined by the open space portion of the microneedle, and draws or wicks physiological sample to within the reaction zone or matrix area of the biosensor. As such, the diameter or width of a single fluid channel or pathway does not exceed 1000 microns and will usually be about 100 to 200 microns in diameter. This diameter may be constant along its length or may vary. It may be preferred that any sub-channels have cross-sectional diameters in the range from about 1 to 200 microns and more usually from about 20 to 50 microns in that they are not required to convey the same volume of fluid as a primary channel.

In certain embodiments of the invention, the fluid pathway may further include one or more agents to facilitate sample collection. For example, one or more hydrophilic agents may be present in the fluid pathway, where such agents include, but are not limited to types of surface modifiers or surfactants such as mercaptoethane sulfonic acid (MESA), Triton, Macol, Tetronic, Silwet, Zonyl, Aerosol, Geropon, Chaps, and Pluronic. In any event, many of the techniques described in U.S. 2002 168 290 and U.S. 2003 212 344 referenced above are applicable to fabricating test strip devices as described herein - especially those details regarding needle/lance production Details as to electrochemical test strip production may also be appreciated in view of EP-A-1 324 038 and EP-A-1 316 367.

However constructed, as commented on above, test strips used in the present invention advantageously include at least one forward-facing lancing member. In which case, receptacle portions **46** of containers **48** according to the present invention are adapted to receive the same.

More specifically, as illustrated in FIG 3, a given receptacle **46** within a container **48** includes a socket or sheath portion **50** to accept and protect microneedle **4.** Shoulder or ledge sections **52** are provided adjacent socket **50** to support a test strip at its face or shoulder portions **54.** The main body of the test strip is preferably received in a upper sheath or jacket portion **56**.

A transition section **58** is preferably provided above the main body sheath portion. As shown, angled faces **60** define the transition section. Optional transition section **58** may serve as an aid in loading a test strip into a given receptacle and/or as a guide for a meter interface member / electrode pair **18** to capture and extract a test strip from the receptacle. Above the transition section, walls **62** may extend further upwards to an aperture **64**. The walls may further serve to guide a test strip extraction (or insertion) element. To facilitate this, the end **10** of a given test strip opposite needle(s) **4** to be handled (*e.g.*, by a meter extraction element) preferably terminates in a widened portion of receptacle **46,** adjacent faces **60**, or more preferably adjacent the region of walls **62.**

Of course the ultimate shape/configuration of receptacle members within a container **48** according to the invention may vary, especially in a manner complimentary to the form of test strips to be housed within the same. Likewise, the external configuration and closure features of test strip canisters according to the present invention may vary. FIGs 2A and 2B show two variations of closure features usable in the present invention.

In FIG 2A, canister **48** includes a lid **66** rotably attached to the canister by way of an arm **68**, rotably secured at a lower pivot **70**. An inset interface in which a portion of the lid rides within a recess of **72** of the container body **74** may provide an upper rotation interface. An access port **76** is provide in lid **66** which, when aligned with a corresponding aperture **64** of a receptacle portion **46** provides access to a test strip **2.** Otherwise, lid 66 closes-off the various receptacle portions included in canister **48.** Meter 6 is preferably configured to automatically actuate the lid, whether by means of rotating bar **68** or another feature, for example, if lid **66** is alternately secured body **74** by way of a pin and cap/shoulder bolt type arrangement **78** (shown in broken line and with dashed leader to indicate use alternate to the connection approach described above).

Another approach to closing off receptacle portions containing unused test strips is illustrated in FIG 2B. Here, container body **74** is covered by a conventional foil laminate cover **76.** Generally, such a laminate includes aluminum foil. Alternately, cover **76** comprises a water barrier polymer film material alone or in combination with a thin foil material wherein the two are laminated together. Suitable materials include those which are commonly used for pharmaceutical and food packaging applications, such as those disclosed in U.S. Patent Nos. 4,769,261, 6287,612 and 4,678,092. The cover can be laminated to the container body by hot melt adhesive or other energy means, such as, ultrasonic welding, heat sealing, RF, *etc*.

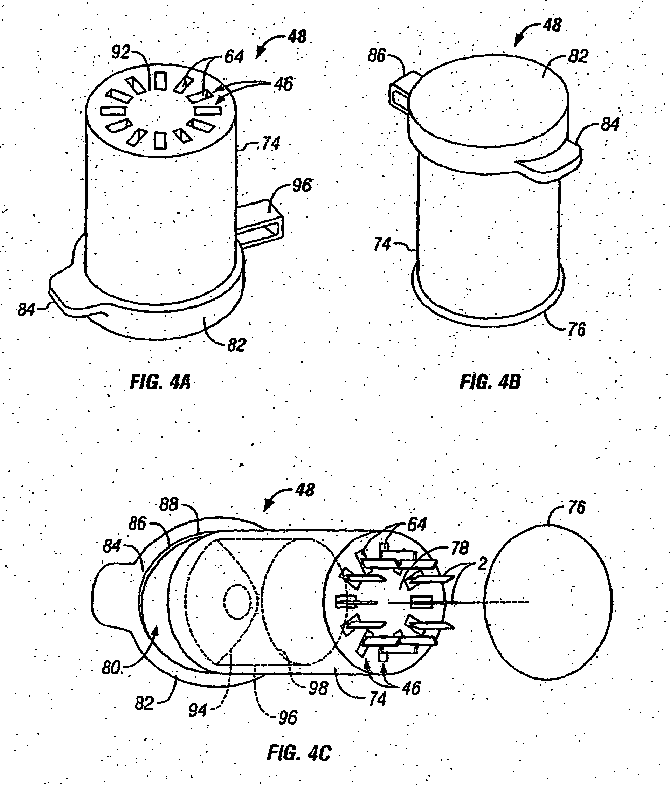

Such a cover is preferably adhered to a face portions **92** of the canister **48**. FIGs 4A and 4B clearly illustrate the face portions between aperture sections **64** at which the connection can be made. FIG 4B shows a foil cover **76** as may be used to cover the plurality of receptacles **46** in canister devices according to the present invention from the underside. When a foil cover is employed, access to each receptacle portion is achieved by puncturing the same at the desired access site by an access member such as interface portion **18** provided in meter **6.**

In some cases, it may be desired, even necessary to provide a canister as configured in FIG 2A with a lin **66** with a supplemental seal or cover **76** as shown in other variations of the invention. In which case, it may provided substantially as described above as well. In any case, such a seal would be located beneath lid **66.**

However access/closure of receptacles **46** is to be provided, it may be preferred to include desiccant in each or one or more adjacent chambers. Suitable desiccants include silica gel beads, molecular sieve, *etc.* Desiccant **90** may simply be deposited and fixed in the end of each receptacle portion or physically entrapped in a separate compartment The desiccant can also be blended into the material that is used to fabricate the receptacles, for instance, in the form of a composite.

Additional optional aspects of the present invention shown in FIGs 4A-4C concern a waste receptacle portion **80** that may integrally be provided in the container **48.** A hollow provided in canister body **48** may be covered by a waste cap or lid **82** to securely house used test strips.

Such strips will generally be ejected from meter 6 into the waste receptacle upon completion of testing. To access the waste section of the container, a user may pop open the lid using extension **84** to provide adequate purchase. A hinge section **86** is preferably provided to maintain association between the canister and its lid. While a simple press-fit or friction fit between the lid interior periphery **86** and container upper, outer periphery **88** is contemplated in the variations shown in FIGs 4A-4C, to form a seal, detent features may be provided. Furthermore, other variations including a latching interface, a threaded interface or the like may be employed to allow opening and closing-off waste receptacle **80**.

The waste container may contain an absorbent material (*e.g.*, a sponge) to soak up any access blood. Waste receptacle **80** may be designed to easily accept the test strip, but make removal of a test strip therefrom difficult. This result can be achieved through geometry of receptacle. For example, a funnel-shaped entry section **94** may be provided. Alternately, a tacky material may be set inside the receptacle, preferably along its internal wall(s) **96** or base **98,** to capture and hold spent test strips. Other means may be employed as well. Providing one-way access to the receptacle for test strips or simply making inadvertent removal/loss of test strips from its confines offers a desirably safety feature.

Each of the containers **48** shown has a generally cylindrical body. Such a configuration is preferred since it facilitates circular disposition of a plurality of receptacles and corresponding number of strip **2** as shown in FIG 4C. The configuration is also highly advantageous from the perspective of automated manipulation of container **48** in indexing (for example in a meter adapted to receive the container/canister) from one receptacle to another containing an unused test strip. However, it is to be appreciated that, just as receptacle and test strip configuration may very, so may that of the container and waste receptacle (when provided). Still, regular shapes, including polygons of up to about 100 sides may be preferred in this regard. The number sides provided may match-up to the number of receptacles/test strips included in a given package.

As to the number of strips and receptacles, it will generally be preferred to include at least 10 pair. However, up to about 100 may be provided. Most preferably between about 20 and 50 receptacles **46,** each containing a single test strip **2** will be provided.

However many are provided, aspects of the present invention require loading and removal of tests strips from the container in a reciprocal fashion. Preferably, they are inserted and removed along the same axis of each test strip. Where a preferred waste receptacle **80** is provided, loading and removing a test strip from the same access port is advantageous because it allows placing the opening of the waste receptacle opposite that of the test strip(s). In instances where forward-facing lance member(s) are provided in the test strip(s), two-way access from the same port **64** allows for the lancet/microneedle protection features described above, referring to sheath or recess section **50**.

In all, the present invention offers numerous potential advantages and configurations. Some variations may enjoy each of these advantages by its various features while others may be more application specific and be best suited to a particular situation.

Though the invention has been described in reference to certain examples, optionally incorporating various features, the invention is not to be limited to the those described. The invention is not limited to the uses noted or by way of the exemplary description provided herein. It is to be understood that the breadth of the present invention is to be limited only by the literal or equitable scope of the following claims.

## Claims (Claims for the following Contracting State(s): DE, FR, GB, IT)

1. A test strip container system comprising:
a plurality of test strips, each including at least one forward facing lancet and face or shoulder portions (54),
a container body (74) defining a plurality of test strip receptacles (46), each receptacle having an access aperture at one end and at least one ledge (52) for supporting a test strip at its face or shoulder portions (54);
a barrier portion (76) for closing off at least some of said access apertures (64) at said one end such that said test strips can be exposed for use one at a time; wherein
each receptacle is configured to receive and protect a lancet (40), each receptacle having:
an inset portion extending from said at least one ledge (52) to form a sheath portion at another end to house and provide clearance for the lancet, and
an access path to the test strip from its rear via said access aperture (64).

2. A test strip container system comprising:
a container body (74), said container body defining a plurality of test strip receptacles (46) at one end and a waste receptacle at another end,
a waste receptacle cap, and
a barrier portion (76) for closing off at least some of said test strip receptacles (46).

3. The system of claim 1 or claim 2, wherein said barrier comprises a foil laminate.

4. The system of claim 1 or claim 2, wherein said barrier comprises a test strip cap.

5. The system of any one of claims 1 to 4, wherein each test strip receptacle includes a transition section from a close-fitting portion for a test strip to said access aperture, wherein said access aperture is enlarged relative to said close-fitting portion.

6. The system of any one of claims 1 or 3 to 5, when not depending on claim 2, further comprising a waste receptacle formed with said container body and a waste receptacle cap.

7. The system of claim 6, wherein a funnel-shaped member provides access to said waste receptacle.

8. The system of any one of the preceding claims, wherein said container body has a substantially cylindrical shape.

9. The system of any one of the preceding claims, wherein said test strip receptacles are arranged in a circular fashion.

10. The system of any one of the preceding claims, wherein each of said test strips includes a biosensor, chosen from electrochemical and colorimetric sensors.

## Claims (Claims for the following Contracting State(s): AT, BE, CH, DK, ES, GR, LI, LU, MC, NL, SE)

1. A test strip container system comprising:
a plurality of test strips, each including at least one forward facing lancet and face or shoulder portions (54),
a container body (74) defining a plurality of test strip receptacles (46), each receptacle having an access aperture at one end and at least one ledge (52) for supporting a test strip at its face or shoulder portions (54);
a barrier portion (76) for closing off at least some of said access apertures (64) at said one end; wherein
each receptacle is configured to receive and protect a lancet (40), each receptacle having:
an inset portion extending from said at least one ledge (52) to form a sheath portion at another end to house and provide clearance for the lancet, and
an access path to the test strip from its rear via said access aperture (64).

2. A test strip container system comprising:
a container body (74), said container body defining a plurality of test strip receptacles (46) at one end and a waste receptacle at another end,
a waste receptacle cap, and
a barrier portion (76) for closing off at least some of said test strip receptacles (46).

3. The system of claim 1 or claim 2, wherein said barrier comprises a foil laminate.

4. The system of claim 1 or claim 2, wherein said barrier comprises a test strip cap.

5. The system of any one of claims 1 to 4, wherein each test strip receptacle includes a transition section from a close-fitting portion for a test strip to said access aperture, wherein said access aperture is enlarged relative to said close-fitting portion.

6. The system of any one of claims I or 3 to 5, when not depending on claim 2, further comprising a waste receptacle formed with said container body and a waste receptacle cap.

7. The system of claim 6, wherein a funnel-shaped member provides access to said waste receptacle.

8. The system of any one of the preceding claims, wherein said container body has a substantially cylindrical shape.

9. The system of any one of the preceding claims, wherein said test strip receptacles are arranged in a circular fashion.

10. The system of any one of the preceding claims, wherein each of said test strips includes a biosensor, chosen from electrochemical and colorimetric sensors.

## Patentansprüche (Patentansprüche für folgende(n) Vertragsstaat(en): DE, FR, GB, IT)

1. Vorratsbehältnissystem für Teststreifen, umfassend:
eine Vielzahl von Teststreifen, wobei jeder zumindest eine nach vorne gerichtete Lanzette und Vorderseiten- oder Schulterabschnitte (54) beinhaltet,
einen Behälterkörper (74), der eine Vielzahl von Teststreifenaufnahmefächern (46) definiert, wobei jedes Aufnahmefach eine Eingangsöffnung an einem Ende und mindestens eine Leiste (52) zum Tragen eines Teststreifens an seinen Vorderseiten- oder Schulterabschnitten (54) aufweist;
einen Barriereabschnitt (76), um zumindest einige der Eingangsöffnungen (64) an dem einen Ende so abzudecken, dass die Teststreifen für die Verwendung jeweils eines Teststreifens freigelegt werden; wobei
jedes Aufnahmefach so konfiguriert ist, dass es eine Lanzette (40) aufnimmt und schützt, wobei jedes Aufnahmefach Folgendes aufweist:
einen Einsatzabschnitt, der sich von der mindestens einen Leiste (52) erstreckt, um am anderen Ende einen Hüllenabschnitt zu bilden, um eine Ausnehmung für die Lanzette zu bilden und diese aufzunehmen, und
einen Zugangsweg zu dem Teststreifen von seiner Rückseite durch die Eingangsöffnung (64).

2. Vorratsbehältnissystem für Teststreifen, umfassend:
einen Behälterkörper (74), wobei der Behälterkörper eine Vielzahl von Teststreifenaufnahmefächern (46) an einem Ende und ein Abfallbehältnis am anderen Ende definiert,
einen Abfallbehälterdeckel, und
einen Barriereabschnitt (76), um zumindest einige der Teststreifenaufnahmefächer (46) abzudecken.

3. System nach Anspruch 1 oder Anspruch 2, wobei die Barriere ein Folienlaminat umfaßt.

4. System nach Anspruch 1 oder Anspruch 2, wobei die Barriere einen Teststreifendeckel umfaßt.

5. System nach einem der Ansprüche 1 bis 4, wobei jedes Teststreifenaufnahmefach einen Übergangsabschnitt von einem an einem Teststreifen eng anlegbaren Bereich zu der Eingangsöffnung enthält, wobei die Eingangsöffnung relativ zu dem eng anlegbaren Bereich vergrößert ist.

6. System nach einem der Ansprüche 1 und 3 bis 5, wenn nicht abhängig von Anspruch 2, ferner umfassend ein Abfallbehältnis, das mit dem Behälterkörper und einem Abfallbehältnisdeckel gebildet wird.

7. System nach Anspruch 6, wobei ein trichterförmiges Element einen Zugang zu dem Abfallbehältnis bereitstellt.

8. System nach einem der vorangegangenen Ansprüche, wobei der Behälterkörper im wesentlichen eine zylindrische Form hat.

9. System nach einem der vorangegangenen Ansprüche, wobei die Teststreifenaufnahmefächer in einer ringförmigen Art und Weise angeordnet sind.

10. System nach einem der vorangegangenen Ansprüche, wobei jeder Teststreifen einen Biosensor, ausgewählt aus elektrochemischen und kolorimetrischen Sensoren, beinhaltet.

## Patentansprüche (Patentansprüche für folgende(n) Vertragsstaat(en): AT, BE, CH, DK, ES, GR, LI, LU, MC, NL, SE)

1. Vorratsbehältnissystem für Teststreifen, umfassend:
eine Vielzahl von Teststreifen, wobei jeder zumindest eine nach vorne gerichtete Lanzette und Vorderseiten- oder Schulterabschnitte (54) beinhaltet,
einen Behälterkörper (74), der eine Vielzahl von Teststreifenaufnahmefächern (46) definiert, wobei jedes Aufnahmefach eine Eingangsöffnung an einem Ende und mindestens eine Leiste (52) zum Tragen eines Teststreifens an seinen Vorderseiten- oder Schulterabschnitten (54) aufweist;
einen Barriereabschnitt (76), um zumindest einige der Eingangsöffnungen (64) an dem einen Ende so abzudecken; wobei
jedes Aufnahmefach so konfiguriert ist, dass es eine Lanzette (40) aufnimmt und schützt, wobei jedes Aufnahmefach Folgendes aufweist:
einen Einsatzabschnitt, der sich von der mindestens einen Leiste (52) erstreckt, um am anderen Ende einen Hüllenabschnitt zu bilden, um eine Ausnehmung für die Lanzette zu bilden und diese aufzunehmen, und
einen Zugangsweg zu dem Teststreifen von seiner Rückseite durch die Eingangsöffnung (64).

2. Vorratsbehältnissystem für Teststreifen, umfassend:
einen Behälterkörper (74), wobei der Behälterkörper eine Vielzahl von Teststreifenaufnahmefächern (46) an einem Ende und ein Abfallbehältnis am anderen Ende definiert,
einen Abfallbehälterdeckel, und
einen Barriereabschnitt (76), um zumindest einige der Teststreifenaufnahmefächer (46) abzudecken.

3. System nach Anspruch 1 oder Anspruch 2, wobei die Barriere ein Folienlaminat umfaßt.

4. System nach Anspruch 1 oder Anspruch 2, wobei die Barriere einen Teststreifendeckel umfaßt.

5. System nach einem der Ansprüche 1 bis 4, wobei jedes Teststreifenaufnahmefach einen Übergangsabschnitt von einem an einem Teststreifen eng anlegbaren Bereich zu der Eingangsöffnung enthält, wobei die Eingangsöffnung relativ zu dem eng anlegbaren Bereich vergrößert ist.

6. System nach einem der Ansprüche 1 und 3 bis 5, wenn nicht abhängig von Anspruch 2, ferner umfassend ein Abfallbehältnis, das mit dem Behälterkörper und einem Abfallbehältnisdeckel gebildet wird.

7. System nach Anspruch 6, wobei ein trichterförmiges Element einen Zugang zu dem Abfallbehältnis bereitstellt.

8. System nach einem der vorangegangenen Ansprüche, wobei der Behälterkörper im wesentlichen eine zylindrische Form hat.

9. System nach einem der vorangegangenen Ansprüche, wobei die Teststreifenaufnahmefächer in einer ringförmigen Art und Weise angeordnet sind.

10. System nach einem der vorangegangenen Ansprüche, wobei jeder Teststreifen einen Biosensor, ausgewählt aus elektrochemischen und kolorimetrischen Sensoren, beinhaltet.

## Revendications (Revendications pour l'(les) Etat(s) contractant(s) suivant(s): DE, FR, GB, IT)

1. Système de récipient pour bandes de test comprenant:
une pluralité de bandes de test, chacune comprenant au moins une lancette tournée vers l'avant et des parties de face ou d'épaulement (54),
un corps de récipient (74) définissant une pluralité de réceptacles (46) pour bandes de test, chaque réceptacle présentant une ouverture d'accès à une extrémité et au moins un rebord (52) pour supporter une bande de test au niveau de ses parties de face ou d'épaulement (54) ;
une partie de barrière (76) destinée à fermer au moins certaines desdites ouvertures d'accès (64) au niveau de ladite une extrémité de telle sorte que lesdites bandes de test puissent être exposées en vue de leur utilisation une à la fois ;
chaque réceptacle étant configuré pour recevoir et protéger une lancette (40), chaque réceptacle rayant :
une partie rentrée s'étendant depuis ledit au moins un rebord (52) pour former une partie de gaine à une autre extrémité pour recevoir la lancette et assurer son dégagement, et
un chemin d'accès pour la bande de test depuis sa partie arrière en passant par ladite ouverture d'accès (64).

2. Système de récipient pour bandes de test comprenant:
un corps de récipient (74), ledit corps de récipient définissant une pluralité de réceptacles (46) pour bandes de test à une extrémité et un réceptacle pour déchets à l'autre extrémité,
un bouchon de réceptacle pour déchets, et
une partie de barrière (76) destinée à fermer au moins certains desdits réceptacles (46) pour bandes de test.

3. Système selon la revendication 1 ou la revendication 2, dans lequel ladite barrière comprend un laminé en feuille.

4. Système selon la revendication 1 ou la revendication 2, dans lequel ladite barrière comprend un bouchon pour bandes de test.

5. Système selon l'une quelconque des revendications 1 à 4, dans lequel chaque réceptacle pour bandes de test comprend une section de transition depuis une partie d'ajustement pour une bande de test jusqu'à ladite ouverture d'accès, dans lequel ladite ouverture d'accès est agrandie par rapport à ladite partie d'ajustement.

6. Système selon l'une quelconque des revendications 1 et 3 à 5, lorsque ne dépendant pas de la revendication 2, comprenant en outre un réceptacle pour déchets formé avec ledit corps de récipient et un bouchon de réceptacle pour déchets.

7. Système selon la revendication 6, dans lequel un élément en forme d'entonnoir donne accès audit réceptacle pour déchets.

8. Système selon l'une quelconque des revendications précédentes, dans lequel ledit corps de récipient a une forme sensiblement cylindrique.

9. Système selon l'une quelconque des revendications précédentes, dans lequel lesdits réceptacles pour bandes de test sont agencés d'une façon circulaire.

10. Système selon l'une quelconque des revendications précédentes, dans lequel chacune desdites bandes de test comprend un biocapteur, choisi parmi des capteurs électrochimiques et colorimétriques.

## Revendications (Revendications pour l'(les) Etat(s) contractant(s) suivant(s): AT, BE, CH, DK, ES, GR, LI, LU, MC, NL, SE)

1. Système de récipient pour bandes de test comprenant:
une pluralité de bandes de test, chacune comprenant au moins une lancette tournée vers l'avant et des parties de face ou d'épaulement (54),
un corps de récipient (74) définissant une pluralité de réceptacles (46) pour bandes de test, chaque réceptacle présentant une ouverture d'accès à une extrémité et au moins un rebord (52) pour supporter une bande de test au niveau de ses parties de face ou d'épaulement (54) ;
une partie de barrière (76) destinée à fermer au moins certaines desdites ouvertures d'accès (64) au niveau de ladite une extrémité ;
chaque réceptacle étant configuré pour recevoir et protéger une lancette (40), chaque réceptacle rayant :
une partie rentrée s'étendant depuis ledit au moins un rebord (52) pour former une partie de gaine à une autre extrémité pour recevoir la lancette et assurer son dégagement, et
un chemin d'accès pour la bande de test depuis sa partie arrière en passant par ladite ouverture d'accès (64).

2. Système de récipient pour bandes de test comprenant:
un corps de récipient (74), ledit corps de récipient définissant une pluralité de réceptacles (46) pour bandes de test à une extrémité et un réceptacle pour déchets à l'autre extrémité,
un bouchon de réceptacle pour déchets, et
une partie de barrière (76) destinée à fermer au moins certains desdits réceptacles (46) pour bandes de test.

3. Système selon la revendication 1 ou la revendication 2, dans lequel ladite barrière comprend un laminé en feuille.

4. Système selon la revendication 1 ou la revendication 2, dans lequel ladite barrière comprend un bouchon pour bandes de test.

5. Système selon l'une quelconque des revendications 1 à 4, dans lequel chaque réceptacle pour bandes de test comprend une section de transition depuis une partie d'ajustement pour une bande de test jusqu'à ladite ouverture d'accès, dans lequel ladite ouverture d'accès est agrandie par rapport à ladite partie d'ajustement.

6. Système selon l'une quelconque des revendications 1 et 3 à 5, lorsque ne dépendant pas de la revendication 2, comprenant en outre un réceptacle pour déchets formé avec ledit corps de récipient et un bouchon de réceptacle pour déchets.

7. Système selon la revendication 6, dans lequel un élément en forme d'entonnoir donne accès audit réceptacle pour déchets.

8. Système selon l'une quelconque des revendications précédentes, dans lequel ledit corps de récipient a une forme sensiblement cylindrique.

9. Système selon l'une quelconque des revendications précédentes, dans lequel lesdits réceptacles pour bandes de test sont agencés d'une façon circulaire.

10. Système selon l'une quelconque des revendications précédentes, dans lequel chacune desdites bandes de test comprend un biocapteur, choisi parmi des capteurs électrochimiques et colorimétriques.
